# EUROPÄISCHE PATENTSCHRIFT

(11) **EP 0 949 253 B1**
(45) Veröffentlichungstag und Bekanntmachung des Hinweises auf die Patenterteilung: **02.01.2003**
(21) Anmeldenummer: 98105578.3
(22) Anmeldetag: 27.03.1998
(51) Int. Cl.: C07D 251/64, C23F 11/14

(54) **Melamin-azomethin-Derivate und ein diese enthaltendes Korrosionsschutzmittel**
Melamine-azomethine derivates and corrosion inhibitor containing these derivates
Dérivés de mélamine-azométhine et inhibiteurs de corrosion les contenant

(43) Veröffentlichungstag der Anmeldung: 13.10.1999
(73) Patentinhaber: Graichen, Stefan, 63546 Hammersbach (DE)
(72) Erfinder: Graichen, Stefan, 63546 Hammersbach (DE)
(74) Vertreter: Meyer-Dulheuer, Karl-Hermann, Dr.

(56) Entgegenhaltungen:
- EP-A- 0 046 139
- EP-A- 0 511 163
- CH-A- 601 999
- DE-A- 1 926 547
- DE-A- 2 606 904
- DE-A- 3 507 751
- US-A- 2 485 309
- PATENT ABSTRACTS OF JAPAN vol. 7, no. 290 (P-245), 24.Dezember 1983 & JP 58 162955 A (CANON KK), 27.September 1983,

## Beschreibung

Gegenstand der Erfindung sind Melamin-azomethin-derivate und ihre Verwendung zur Inhibierung der Korrosion von Eisen oder eisenhaltigen Metallen, die in Kontakt mit wässrigen Systemen stehen.

Es sind bereits eine Reihe von Melamin-azomethinderivaten aus der Schweizer Patentschrift 601 999, den deutschen Patentanmeldungen 35 07 751 und 26 06 904 sowie aus der japanischen Patentanmeldung 58 1622955 bekannt, die als Herbizide, Pigmente und Stabilisatoren in photosensitiven Schichten Anwendung finden. Es ist außerdem aus der deutschen Patentanmeldung 19 26 547 bekannt, dass bestimmte heterozyklische Azomethderivate die Beständigkeit von Olefinpolymerisaten gegen den durch Kupfer katalysierten oxidativen Abbau erhöhen.

Es ist bekannt, dass erhebliche Anstrengungen unternommen werden, um die Korrosion von Metallen zu vermindern. So ist in der europäischen Patentschrift 46 139 die Verwendung von Triazincarbonsäuren als Korrosionsinhibitoren für wässrige Systeme, die in Kontakt mit Eisen oder eisenhaltigen Metallen stehen, vorgeschlagen worden. Aus der europäischen Patentschrift 511 163 sind fließfähige wässrige Dispersionen bekannt, die einen festen Polycarbonsäuretriazin-Korrosionsinhibitor enthalten. Weiterhin ist aus der US-A-4 402 907 und der EP-A-129 5096 bekannt, dass bestimmte heterozyklische Polycarbonsäuren als Korrosionsinhibitoren für wässrige Systeme die mit Metallen in Kontakt stehen, gut geeignet sind. Sie können in wässrigen Systemen, zum Beispiel in Kühlwassersystemen, in Dampferzeugungsanlagen, in Metallbearbeitungsmitteln und wässrigen Hydraulikflüssigkeiten verwendet werden. Da die meisten Polycarbonsäuren in Wasser nur eine geringe Löslichkeit haben, verwendet man die Polycarbonsäuren in Form ihrer wasserlöslichen Salze, d.h., man neutralisiert sie vor dem Gebrauch oder man setzt sie einem basischen Wassersystem zu. Lager- und Handelsform sind jedoch im allgemeinen die freien Polycarbonsäuren. Die freien Polycarbonsäuren sind im allgemeinen feste Stoffe. Bei ihrer Herstellung werden sie meist durch Filtration aus einer wäßrigen Phase isoliert. Das filtrierte Produkt wird üblicherweise mit Wasser gewaschen und dann einer Trocknung unterzogen. Um die für die Trocknung benötigte Energie zu sparen, hat man neuerdings für die Verwendung in wäßrigen Systemen den feuchten Filterkuchen, der etwa 50% Wasser enthält, als Handelsform angeboten. Der feuchte Filterkuchen hat jedoch den Nachteil, daß er nicht fließfähig ist. Er kann nicht geschüttet oder gegossen werden, sondern wird mit der Schaufel manuell dosiert oder umgefüllt. Deshalb ist auch schon vorgeschlagen worden, Polycarbonsäuren in Form von hochprozentrigen wäßrigen Dispersionen einzusetzen.

Auch auf der Basis von Melamin entwickelte Korrosionsinhibitoren sind bereits bekannt. So werden in der US-A-2 485 309 Methylolmelamin-Kondensationsprodukte beschrieben, die aber den hohen Anforderungen, welche heute an ein Korrosionsschutzmittel gestellt werden, keinesfalls genügen.

Es wurde nun gefunden, daß neuartige Melamin-azomethin-derivate und ihre wasserlöslichen Salze ausgezeichnete Korrosionsinhibitoren in wäßrigen Systemen sind und sich zum Einsatz in Wasserkreisläufen, in wäßrigen Maschinenflüssigkeiten, Gefrierschutzflüssigkeiten, hydraulischen Flüssigkeiten oder wäßrigen Anstrichmitteln hervorragend eignen.

Gegenstand der Erfindung sind deshalb Melamin-azomethin-derivate der Formel I, 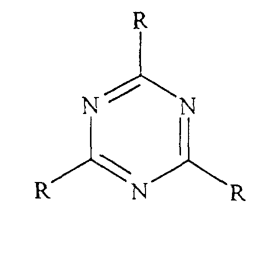 in der mindestens einer der Reste R ein Substituent der Formel II

- N = CZ - X - Y

ist, in der
- Z Wasserstoff oder Methyl,
- Y eine COOH- oder eine -CHO-Gruppe, die mit einem weiteren Melaminrest kondensiert sein kann, oder eine -OH-Gruppe und
- X ganz entfallen kann oder einen geradkettigen oder verzweigten Alkylen- oder Alkenylenrest mit bis zu 12 Kohlenstoffatomen bedeutet, der auch Hydroxylgruppen tragen und einen Cyclopentylen-, Cyclohexylen- oder einen Phenylenrest enthalten kann, sowie ihre Alkali-, Ammonium- und Aminsalze.

Die erfindungsgemäßen Melamin-azomethin-derivate werden durch Kondensation einer NH₂-Gruppe des Melamins mit einer gesättigten oder ungesättigten, geradkettigen oder verzweigten Aldehyd- oder Ketocarbonsäure, einem Dialdehyd oder einer Aldose gebildet. Als Aldehydcarbonsäure kann zum Beispiel die Glyoxylsäure, die 3-Oxopropansäure, die 4-Oxobutansäure, die 5-Oxopentansäure, die Glukuronsäure, die Phthalaldehydsäure oder die Terephthalaldeyhdsäure eingesetzt werden. Als Ketocarbonsäuren kann z.B. die 3-Oxoglutarsäure oder die Lävulinsäure eingesetzt werden.

Als Dialdehyde haben sich Glyoxal und Succindialdehyd zur Herstellung der erfindungsgemäßen Melamin-azomethin-derivate geeignet erwiesen. Dabei verküpft ein Dialdehyd zwei Melaminmoleküle durch zweifache Azomethinbildung miteinander. Die freien Aminogruppen des Reaktionsproduktes bilden dann mit weiteren Dialdehydmolekülen dendridartige Strukturen, die eine deutliche Erhöhung der Viskosität der Reaktionslösung zur Folge haben.

Die erfindungsgemäßen Melamin-azomethin-derivate können auch durch Reaktion von Aldosen mit Melamin gebildet werden. Geeignet hierfür sind Tetrosen, Pentosen und Hexosen, ganz besonders aber Glukose.

Besonders bevorzugt sind Melamin-azomethin-derivate, bei denen alle drei Aminogruppen des Melamins sich mit der Aldehydgruppe der Carbonsäure unter Azomethinbildung umgesetzt haben. Es können jedoch auch Melamincarbonsäuren verwendet werden, bei denen nur eine oder zwei Aminogruppen des Melamins mit der Aldehydgruppe der Aldehydcarbonsäure unter Azomethinbildung reagiert haben. Die zur Reaktion mit dem Melamin verwendeten Aldehydcarbonsäuren können in Molverhältnissen von 1 : 1 bis 1 : 3 eingesetzt werden, wobei in Abhängigkeit von den eingesetzten Mengenverhältnissen unterschiedliche Melaminazomethin-derivatgemische entstehen. Die sich dabei bildenden Reaktionsprodukte weisen freie Carbonsäuren auf, die bei Anwendung eines Überschusses an Melamin mit einem weiteren Melaminrest kondensieren können. Es sollten jedoch mindestens 80%, vorzugsweise mehr als 90% der Carbonsäuren noch als freie Carbonsäuren vorliegen, damit sich die erfindungsgemäßen Melamin-azomethin-derivate im alkalischen Medium und unter Salzbildung in Wasser lösen können.

Die Herstellung der Melamin-azomethin-derivate kann dadurch erfolgen, daß die durch Alkalizugabe neutralisierte Aldehydcarbonsäure in wäßriger Lösung bei 70 bis 80°C langsam mit dem Melamin versetzt wird. Dabei löst sich das Melamin unter Rühren im Laufe von 10 bis 30 Minuten auf. Im allgemeinen wird die Aldehydcarbonsäure in der dreifach molaren Menge, bezogen auf das Melamin, eingesetzt. Es ist jedoch durchaus auch möglich, größere Mengen Melamin zu verwenden, so daß nicht alle Aminogruppen des Melamins mit einer Aldehydgruppe unter Azomethinbildung reagieren können. Nach Beendigung der Reaktion wird die wäßrige Lösung filtriert und das Filtrat zur Trocknung eingedampft. Je nach dem eingesetzten Alkalisierungsmittel liegt dann die erfindungsgemäße Melamin-azomethin-derivate als Alkali-, Ammonium- oder Aminsalz, zum Beispiel als Salz des Triethanolamins vor.

Die für die Herstellung der erfindungsgemäßen Melamin-azomethin-derivate erforderlichen Aldehydcarbonsäuren können auch aus geeigneten Vorstufen durch Oxidations- oder Reduktionsvorgänge unmittelbar vor der Umsetzung mit dem Melamin hergestellt werden.

So kann beispielsweise ausgehend von Glyoxal nach einem an sich bekannten Oxidationsverfahren zunächst Glyoxylsäure hergestellt und diese dann ohne vorherige Isolierung zur Umsetzung mit dem Melamin verwendet werden. Andererseits kann aber auch eine Dicarbonsäure wie die Oxalsäure oder die Terephthalsäure durch eine katalytische Reduktion zur entsprechenden Aldehydcarbonsäure umgewandelt werden.

Die erfindungsgemäßen Melamin-azomethin-derivate können als solche als Korrosionsschutzmittel eingesetzt werden, sie können aber auch durch Hydrierung der Azomethin-Doppelbindung in die aus dem europäischen Patent 0 046 139 bekannten Triazincarbonsäuren umgewandelt werden, die als Korrosionsinhibitoren für wäßrige Systeme schon bekannt sind. Die dort beschriebenen Triazincarbonsäuren wurden bisher durch Umsetzung von Cyanursäurechlorid mit einem Amin oder Aminocarbonsäure unter Abspaltung von Chlorwasserstoff hergestellt. Dieser muß mit Alkali neutralisiert und anschließend aus dem Reaktionssystem entfernt werden. Demgegenüber bietet die erfindungsgemäße Hydrierung von Melamin-azomethin-derivate den Vorteil, daß die als Korrosionsschutzmittel einsetzbaren Melamincarbonsäuren ohne Nebenprodukte erzeugt werden.

Die Melamin-azomethin-derivate werden vorzugsweise in einer Menge von 0,001 bis 5 Gewichtsprozent, bezogen auf das wäßrige System, verwendet. Bei dem wäßrigen System kann es sich um Wasserkreisläufe, beispielsweise Kühlwasserkreisläufe, Kreisläufe von wäßrigen Maschinenflüssigkeiten wie beispielsweise Kühlflüssigkeiten beim Bohren, Mahlen, Fräsen, Drehen, Schneiden, Sägen, Schleifen, Gewindeschneiden oder beim Walzen oder Ziehen von Metallen handeln. Auch Gefrierschutzmittel oder hydraulische Flüssigkeiten auf Glykol-Wasserbasis sowie wäßrige Anstrichfarben, zum Beispiel Dispersionsfarben oder wäßrige Pulverlacke können mit den erfindungsgemäßen Melamin-azomethin-derivate antikorrosiv ausgerüstet werden.

Die Melamin-azomethin-derivate können in den wäßrigen Systemen als alleiniger Zusatz oder in Kombination mit anderen Zusätzen verwendet werden. Beispiele für solche Co-Additive in Wasserkreisläufen sind bekannte Korrosionsinhibitoren wie Phosphonate, Phosphonocarbonsäuren oder Phosphinocarbonsäuren, N-Acylsarkosine, Imidazoline, Triethanolamin, Fettamine oder Polycarbonsäuren. Diese können Kupfer-Passivatoren sein wie wasserlösliche Benztriazole, Methylen-bis-benztriazole oder 2-Mercaptobenzthiazole. Weiterhin können Dispersionsmittel und Trägerstoffe wie Poly(meth)acrylsäure und ihre Salz, hydrolisiertes Polyacrylnitril, Polyacrylamid und dessen Copolymere, Ligninsulfonsäure und deren Salze, Stärke und Stärkecarbonsäuren, Cellulose, Alkylphosphonsäuren, 1-Aminoalkyl-1,1-diphosphonsäuren und ihre Salze, Polymaleinsäuren und andere Polycarbonsäuren oder Alkaliphosphate zugegeben werden.

Weitere Co-Additive können Fällungsmittel sein, wie Alkaliphosphate oder Alkalicarbonate, Sauerstoffabfänger wie Alkalisulfate oder Hydrazin, Komplexierungsmittel wie Nitrilotriessigsäure oder Ethylendiamin-tetraessigsäure und deren Salze, oder schaumverhütende Mittel wie Distearylsebacinsäurediamid, Distearyladipinsäurediamid oder Ethylenoxidoder Propylenoxid-Kondensationsprodukte solcher Amide, sowie Fettalkohole und deren Ethylenoxid-Kondensationsprodukte.

wäßrige Systeme, die als Maschinenflüssigkeiten verwendet werden, können ein wasserverdünnbares Schneid- oder Schleiföl sein, wie
a) wäßrige Konzentrate eines erfindungsgemäßen Melaminpolycarbonsäureamids mit oder ohne einen Antiverschleißzusatz, die dann in einer Verdünnung von 1 : 50 bis 1 : 100 als Schleifflüssigkeit verwendet werden können,
b) Polyglykole, die ein Melamin-polycarbonsäureamid, Biocide, Korrosionsinhibitoren und Antiverschleißmittel enthalten und die als Schneidflüssigkeit in einer Verdünnung von 1 : 20 bis 1 : 40 und als Schleifflüssigkeit in einer Verdünnung von 1 : 60 bis 1 : 80 verwendet werden können,
c) halbsynthetische Schneidöle auf ähnlicher Basis wie b), jedoch zusätzlich 10-25% eines Öls enthaltend sowie genügend Emulgator, um die Flüssigkeit beim Verdünnen transparent zu halten,
d) emulgierbare Mineralöl-Konzentrate, die außer dem Emulgator ein Melamin-azomethin-derivat, Antiverschleißmittel, Biocide und Antischaummittel enthalten können und die üblicherweise im Verhältnis 1 : 20 bis 1 : 50 mit Wasser zu einer opaken Emulsion verdünnt werden,
e) Produkte ähnlich d), jedoch weniger Öl und mehr Emulgator enthaltend, die bei einer Verdünnung von 1 : 50 bis 1 : 100 durchscheinende Emulsionen ergeben.

Auch in Gefrierschutzmittel oder Hydraulikflüssigkeiten können die Melamin-azomethin-derivate entweder allein oder in Kombination mit anderen Zusätzen verwendet werden. Zusätzlich können darin auch andere Korrosionsinhibitoren enthalten sein, wie
a) organische Säuren, deren Salze und Ester, zum Beispiel Benzoesäure, p-tert.Butylbenzoesäure, Dinatrium-sebacat, Triethanolamin-laurat, Isononansäure, das Triethanolaminsalz der p-Toluolsulfonamido-capronsäure, Natrium-N-lauroyisarcosinat oder Nonylphenoxyessigsäure;
b) stickstoffhaltige Substanzen, wie Fettsäurealkanolamide, Imidazoline, Oxazoline, Triazole oder anorganische Nitrite oder Nitrate;
c) phosphorhaltige Substanzen, beispielsweise Aminphosphate, Phosphonsäuren oder anorganische Phosphate, wie NaH₂PO₄;
d) schwefelhaltige Substanzen, beispielsweise Salze von Petroleumsulfonaten, oder heterocyclische Verbindungen wie Natrium-mercaptobenzthiazol.

Die erfindungsgemäßen Melamin-azomethin-derivate können in einer wäßrig-alkalischen Lösung mit einem pH-Wert über 8,0 oder auch als fließfähige wäßrige Dispersionen eingesetzt werden. Als Dispergiermittel eignen sich alle oberflächenaktiven Verbindungen, insbesondere anionische und nichtionische Tenside. Derartige Dispersionen können durch Verdickungsmittel stabilisiert werden, wobei man als Stabilisatoren vor allem modifizierte Polysaccharide vom Xanthan-, Alginat-, Guar- oder Cellulose-Typ verwendet. Dazu gehören auch Celluloseäther, wie Methylcellulose oder Carboxylmethylcellulose und Heteropolysaccharide. Außer dem Dispergiermittel und dem Verdickungsmittel können die erfindungsgemäßen Dispersionen noch weitere Hilfsmittel enthalten, beispielsweise hydrotrope Mittel wie Harnstoff oder Natriumxylolsulfonat; Gefrierschutzmittel wie Ethylen- oder Propylenglykol, Diethylenglykol, Glycerin oder Sorbit; Biozide wie Chloracetamid, Formalin oder 1-2-Benzisothiazolin-3-on oder Komplexbildner. Zur Herstellung der Dispersionen geht man zweckmäßig von einer festen Melamin-azomethin-derivate aus, setzt dieser das Dispergiermittel und das Verdickungsmittel sowie ggf. die gewünschte Menge Wasser und weitere Zusätze zu, und rührt das Gemisch solange, bis eine fließfähige homogene Dispersion entstanden ist. Die so hergestellten Dispersionen sind bei Raumtemperatur sowie bei Temperaturen bis zu 40°C mehrere Monate stabil. Sie behalten ihre Fließfähigkeit und entmischen sich nicht. Das ist für die Lagerung und den Transport der Dispersionen eine wichtige Eigenschaft. Für die Verwendung der Dispersionen ist es von Vorteil, daß sie wie Flüssigkeiten gehandhabt werden können und sich sehr schnell in alkalisch-wäßrigen Systemen lösen.

Die Erfindung wird durch die folgenden Beispiele näher veranschaulicht:

### Beispiel 1

In einem mit Rührer versehenen Reaktionsbehälter werden 3 Mol Glyoxylsäure und 3 Mol Triethanolamin in Wasser gelöst und auf 70 bis 80°C erwärmt. Dann wird 1 Mol Melamin langsam eingerührt und 30 Minuten weiter erhitzt. Dabei löst sich das Melamin auf. Nach Abfiltrieren kann die Lösung nach Zugabe von weiterem Triethanolamin als Puffer direkt als Korrosionsschutzmittel verwendet werden, indem sie in einer Menge von 3% einer Schleifflüssigkeit, einer Kühlerflüssigkeit oder einem Frostschutzmittel eines Korrosionsinhibitor zugesetzt wird.

### Beispiel 2

In einem mit Rührer versehenen Reaktionsbehälter werden 1 Mol Melamin und 3 Mol Glukose in 500 bis 1000 ml Wasser bei etwa 80°C umgesetzt. Nach 20 min hat sich das Melamin vollständig gelöst. Das Reaktionsprodukt kann entweder als solches oder nach Oxidation zur entsprechenden Carbonsäure als Korrosionsschutzmittel eingesetzt werden.

### Beispiel 3

In einem mit Rührer versehenen Reaktionsbehälter werden 1 Mol Melamin und 3 Mol Glyoxal in 500 bis 1000 ml Wasser bei etwa 80°C umgesetzt. Bei Zugabe eines Oxidationsmittels wie H₂O₂ oder HNO₃ lösen sich die Ausgangsstoffe und es entsteht ein Carboxylgruppen enthaltendes Produkt, das sich als Korrosionschutzmittel hervorragend eignet.

### Beispiel 4

Die Bestimmung der erfindungsgemäßen Korrosionsschutzeigenschaften von Melamin-azomethin-derivate erfolgt gemäß der Vorschrift der DIN 51360 Teil II. Als Maß der Korrosion dienen dabei die Korrosionszeichnungen auf einem Rundfilterpapier, die bei der Einwirkung von künstlich korrosiv gemachtem Wasser auf Graugußspäne in Gegenwart der Melamin-azomethin-derivate entstehen. Der Versuch wird dabei in folgender Weise durchgeführt:
1. 1 Mol Melamin wird mit 1, 2 oder 3 Mol einer Aldehydcarbonsäure in wäßriger Lösung umgesetzt, bis die Kondensationsreaktion beendet ist. Dann wird das verwendete Lösungsmittel abdestilliert. Die erhaltene Melamin-azomethin-derivat ist zu 8% bzw. zu 10% in einem Korrosionsschutzmittel enthalten, das außerdem noch aus 60% Triethanolamin rein (85%) und Leitungswasser besteht.
2. Entsprechend der Vorschrift der DIN 51360, Teil II wird nun in ein Becherglas Wasser mit einer Gesamthärte von 3,58 mmol, hergestellt aus CaCl₂ x 6H₂O und MgSo₄ x 7H₂O, mit 3% des oben genannten Korrosionsschutzmittels versetzt und mit dieser Lösung die in einer Petrischale auf Rundfilter verteilten Graugußspäne benetzt. Nach einer Verweilzeit von 2 Stunden bei Zimmertemperatur werden die Graugußspäne vom Rundfilter entfernt, das Rundfilter gespült und getrocknet und dann der Korrosionsgrad visuell entsprechend der Tabelle der DIN 51360, Teil II bestimmt.

Dabei wurden folgende Ergebnisse erhalten:

| **Korrosionsgrade der Melamin-azomethin-derivate** | | | | |
|---|---|---|---|---|
| **Versuchs- Nr.** | **Melamin-azomethin- derivat aus** | **pH einer 3%- igen Lösung des Korrosionsschutzmittels** | **Korrosionsgrad (8%-Azomethin)** | **Korrosionsgrad (12%-Azomethin)** |
| 1 | 1 Mol Melamin + 3 Mol Glyoxylsäure | 8,75 | 3 | 1 |
| 2 | 1 Mol Melamin + 3 Mol Terephthalaldehydsaure | 9,0 | 2-3 | 1 |
| 3 | Terephthalaldehydsäure (Vergleich) | 8,3 | 4 | 4 |
| 4 | 1 Mol Melamin + 2 Mol Glukuronsäure | 8,75 | 2-3 | 2 |
| 5 | 1 Mol Melamin + 3 Mol 4-Oxobutansäure | 8,2 | 2-3 | 1 |
| 6 | 1 Mol Melamin + 3 Mol 3-Oxopropansäure | 8,65 | 2 | 1 |

Die Tabelle zeigt, daß gute antikorrosive Eigenschaften mit allen erfindungsgemäßen Melamin-azomethin-derivate erhalten werden können, die sich noch verbessern lassen, wenn die Melamin-azomethin-derivate im Korrosionsschutzmittel in einer höheren Konzentration, zum Beispiel zu 10%, enthalten ist.

## Patentansprüche

1. Melamin-azomethin-derivat der Formel I 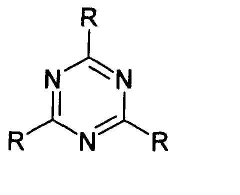 in der mindestens einer der Reste R ein Substituent der Formel II
-N = CZ - X - Y
ist und die übrigen Reste R Aminogruppen sind, wobei in der Formel II
- Z Wasserstoff oder Methyl,
- Y eine COOH- oder eine -CHO-Gruppe, die mit einem weiteren Melaminrest kondensiert sein kann, oder eine -OH-Gruppe und
- X ganz entfallen kann oder einen geradkettigen oder verzweigten Alkylen- oder Alkenylenrest mit bis zu 12 Kohlenstoffatomen bedeutet, der auch Hydroxylgruppen tragen und einen Cyclopentylen-, Cyclohexylen- oder einen Phenylenring enthalten kann, sowie seine Alkali-, Ammonium- und Aminsalze.

2. Melamin-azomethin-derivat nach Anspruch 1, **dadurch gekennzeichnet, dass** es aus Melamin und einer Aldehydcarbonsäure im Molverhältnis 1 : 1 bis 1 : 3 hergestellt ist.

3. Korrosionsschutzmittel, für Eisen oder eisenhaltige Metalle, welche sich im Kontakt mit wässrigen Systemen befinden, **dadurch gekennzeichnet, dass** es ein Melamin-azomethin-derivat oder ein Melamin-azomethin-derivatgemisch gemäß den Ansprüchen 1 oder 2 enthält.

4. Verwendung eines Melamin-azomethin-derivates oder eines Melamin-azomethin-derivatgemisches gemäß den Ansprüchen 1 oder 2 zur Herstellung von als Korrosionsschutzmittel einsetzbaren Melamincarbonsäuren durch Hydrierung.

## Claims

1. Melamine-azomethine derivative of the formula I 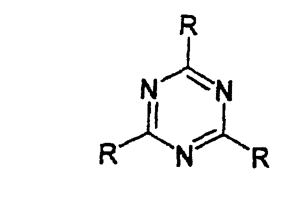 in which at least one of the radicals R is a substituent of the formula II
-N = CZ - X - Y
and the other radicals R are amino groups, in the formula II
- Z denoting hydrogen or methyl,
- Y denoting a COOH- or a -CHO group, which may be condensed with a further melamine radical, or an -OH group and
- It being possible for X to be completely ommitted or to denote a straight-chain or branched alkylene or alkenylene radical having up to 12 carbon atoms, which may also carry hydroxyl groups and may contain a cyclopentylene, cyclohexylene or phenylene ring, and its alkali metal, ammonium and amine salts.

2. Melamine-azomethine derivative according to Claim 1, **characterized in that** it is prepared from melamine and an aldehydecarboxylic acid in a molar ratio of from 1 : 1 to 1 : 3.

3. Corrosion inhibitor for iron or iron-containing metals which are in contact with aqueous systems, **characterized in that** it contains a melamine-azomethine derivative or a melamine-azomethine derivative mixture according to either of Claims 1 and 2.

4. Use of a melamine-azomethine derivative or of a melamine-azomethine derivative mixture according to either of Claims 1 and 2 for the preparation, by hydrogenation, of melaminecarboxylic acids which can be used as corrosion inhibitors.

## Revendications

1. Dérivé de mélamine-azométhine de formule I 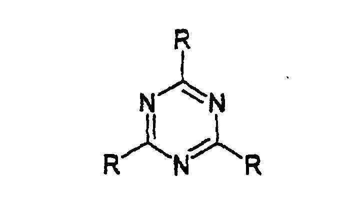 dans laquelle l'un des radicaux R est un substituant de formule II
-N=CZ-X-Y
et les autres radicaux R sont des groupes amino, où dans la formule II
- Z représente un hydrogène ou un méthyle,
- Y représente un groupe COOH- ou -CHO, qui peut être condensé avec un radical mélamine supplémentaire, ou un groupe -OH et
- X peut être entièrement supprimé ou représente un radical alkylène ou alcénylène linéaire ou ramifié ayant jusqu'à 12 atomes de carbone, qui peut également porter des groupes hydroxy et renfermer un noyau cyclopentylène, cyclohexylène ou phénylène, et ses sels de métaux alcalins, d'ammonium et d'amines.

2. Dérivé mélamine-azométhine selon la revendication 1, **caractérisé en ce qu'**il est préparé à partir de la mélamine et d'un acide aldéhyde carboxylique en un rapport molaire de 1:1 à 1:3.

3. Agent de protection anti-corrosive pour le fer ou des métaux contenant du fer, qui se trouvent en contact avec des systèmes aqueux, **caractérisé en ce qu'**il comprend un dérivé mélamine-azométhine ou un mélange de dérivés mélamine-azométhine selon les revendications 1 ou 2.

4. Utilisation d'un dérivé mélamine-azométhine ou d'un mélange de dérivés mélamine-azométhine selon les revendications 1 ou 2 pour la préparation d'acides mélaminecarboxyliques utilisables en tant qu'agent de protection anti-corrosive par hydrogénation.
